# EUROPEAN PATENT SPECIFICATION

(11) **EP 1 462 053 B1**
(45) Date of publication and mention of the grant of the patent: **12.03.2014**
(21) Application number: 04251802.7
(22) Date of filing: 26.03.2004
(51) Int. Cl.: A61B 5/15

(54) **An improved lance with two elements**
Verbesserte Lanzette mit zwei Elementen
Lancette ameliorée avec deux éléments

(30) Priority: 28.03.2003 US 459465 P; 11.06.2003 US 460106
(43) Date of publication of application: 29.09.2004
(73) Proprietor: LifeScan, Inc., Milpitas, CA 95035 (US)
(72) Inventor: Allen, John J., Mendota Heights, MN 55118 (US)
(74) Representative: Mercer, Christopher Paul

(56) References cited:
- WO-A-02/100461
- WO-A-2004/041087
- US-A- 2 896 628
- US-A- 3 143 793
- US-A1- 2002 177 761

## Description

### BACKGROUND OF THE INVENTION

### Field of the Invention

The present invention relates, in general, to lancing elements for use in drawing bodily fluids out of a patient and, more particularly, to an improved lancing element including first and second elements positioned relative to each other such that an incision formed by the first element is held open by the second element and bodily fluids are pulled up the lancing element by surface tension on the first and second lancing elements.

### Description of the Related Art

Integrated skin lancing and body fluid analysis samplers are known in the art. One such system is described and illustrated in WO 02/49507. The integrated system described in WO 02/49507 includes a lancing element or lance, which is attached to or integrated with a test strip adapted to measure the quantity of an analyte in bodily fluid or, alternatively, some characteristic of the bodily fluid. Usable bodily fluids may include, for example, blood or interstitial fluid (ISF). The lancing element is used to make an incision in the skin and the bodily fluid is drawn up the lancing element to the test strip by, for example, capillary action. Such integrated samplers may be combined with, for example, an electrochemical meter and referred to as monolithic or in-situ sampling devices.

Many lancing devices have been devised to form incisions and to enable bodily fluids to be withdrawn from those incisions. Solid lancets are used to open an incision in the skin to allow bodily fluids to escape to the surface of the skin where they can be sampled by the patient or the doctor. An example of such a lancing device is disclosed in US 2,896,628. In order to ensure that enough fluid is released from the incision, such solid lancing elements are generally larger in diameter to facilitate the flow of sufficient bodily fluids from the incision for sampling purposes. However, such solid needles generally rely on the size of the incision to ensure that enough bodily fluids are expressed and are not used to facilitate the flow of fluids to the testing apparatus.

Hollow needles have also been described for use in drawing fluids out of the body for testing purposes; such needles may have a pointed or beveled end to facilitate opening the incision. An example of such a hollow needle is disclosed in WO 02/100461 A2. In such needles, the incision is held open by the outer diameter of the needle to facilitate the flow of bodily fluids out of the incision and the bodily fluids are drawn up the needle either by a vacuum or by capillary action or by a combination of vacuum and capillary action.

Other lancing devices have been described wherein the lance is a flat or partially curved piece which includes an open channel for guiding fluid from the sharpened tip to the proximal end of the lance by means of, for example, surface tension and/or capillary action. Such lacing elements are advantageous because of the ease of manufacture and the ease of integrating them into, for example, a test strip, in order to facilitate both lancing and measurement in a single element. Where the landing element is a flat or partially flat piece which includes an open channel for guiding fluid, it is possible for the edges of the incision to close on the channel, fully or partially blocking the channel and preventing bodily fluids from flowing to the proximal end of the channel or limiting the amount of fluid which can flow.

### Problem to be Solved

It would, therefore, be advantageous to design a lancing device where the lancing element is a flat or partially curved piece including an open channel and the lancing element includes a separation element for holding the incision open when the lancing element is in the wound and preventing the edges of the incision from closing on the lancing element and partially or fully blocking the open channel. It would be advantageous to design a lancing device wherein the separation element is positioned slightly proximal to the sharpened tip of the lancing element to facilitate insertion of the lance into the skin. It would further be advantageous to design a lancing device wherein the lancing element and the separation element are formed from a single metal sheet. It would further be advantageous to design a lancing device wherein the lancing element and the separation element are positioned opposite each other such that fluid is pulled up the lancing element and into the open channel by surface tension between the fluid and the lancing element and separation element, thus facilitating the filling of the channel. It would further be advantageous to design a lancing device wherein the lancing element and the separation element are formed from a single sheet of metal rolled to position the separation element opposite the lancing element such that the proximal end of the lancing element and the separation element form an open channel. It would further be advantageous to manufacture the lancing devices described herein using, for example, a metal forming or stamping process.

### SUMMARY OF THE INVENTION

The present invention provides a lance in accordance with the appended claim 1.

A method of lancing skin may include the step of providing an appropriate lance. An appropriate lance in accordance with the present invention may include a lancing element having a first sharpened end point, a separation element having a second end point positioned adjacent the lancing element, a connector, connecting a proximal portion of the first lancing element to a proximal portion of the separation element, the connector forming a channel. At least one of the separation element, lancing element and channel comprises either (i) a surfactant coating, or (ii) a hydrophilic surface treament. In a method for using a lance according to the present invention, the steps may include inserting the lancing element to form an incision, inserting the separation element to further open the incision, maintaining the lancing elements and the separation element in the incision while bodily fluids are drawn into a space between the lancing elements. The steps may further include the step of drawing the bodily fluids from the space into the channel.

### BRIEF DESCRIPTION OF THE DRAWINGS

While the novel features of the invention are set forth with particularity in the appended claims, a better understanding of the features and advantages of the present invention will be obtained by reference to the following detailed description that sets forth illustrative embodiments, in which the principles of the invention are utilized, and the accompanying drawings of which:
Figure 1 is a perspective view of a lancing element and strip according to the present invention.
Figure 2 is a perspective view of the top layer of a lancing element and strip according to the present invention.
Figure 3 is a perspective view of another embodiment of the invention in which multiple strips form an array of sensors for use in a cartridge format.

### DETAILED DESCRIPTION OF ILLUSTRATIVE EMBODIMENTS OF THE INVENTION

| ELEMENTS | |
|---|---|
| 10 | 1st electrode contact |
| 11 | Adhesive layer |
| 12 | Conductive substrate |
| 13 | Vent hole |
| 15 | Lance |
| 17 | 2^{nd} electrode contact |
| 18 | Insulating substrate |
| 37 | Reference electrode |
| 20 | Insulating layer |
| 21 | Fill channel |
| 22 | Lancing element |
| 23 | Registration hole |
| 24 | Separation element |
| 31 | Index hole |
| 32 | Neck |
| 33 | Contact hole |
| 36 | Working electrode |
| 38 | Sharpened tip |
| 40 | Separation tip |
| 42 | Gap |
| 100 | Sensor strip |

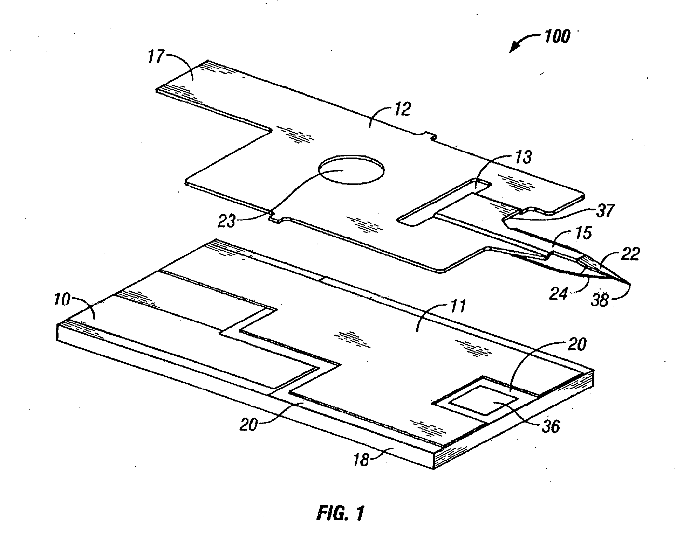

Figure 1 is a perspective view of lance 15 and sensor strip 100 according to the present invention. In Figure 1, lance 15 is connected to sensor strip 100. Sensor strip 100 may be, for example, a glucose sensor strip which uses electrochemistry to measure the amount of glucose in a bodily fluid, such as, for example, blood or interstitial fluid. Additionally, sensor strip 100 may be, for example, a coagulation sensor which measures a physical characteristic of a body fluid such as viscosity, capacitance, resistance, and the like. In Figure 1, lance 15 further includes lancing element 22 and separation element 24. Sensor strip 100 further includes first electrode contact 10, adhesive layer 11, conductive substrate 12, vent hole 13, second electrode contact 17, insulating substrate 18, insulating layer 20, registration hole 23 and working electrode 36. In an embodiment of the invention, sensor strip 100 may have an approximate width of 0.22 inches and an approximate length of 0.55 inches.

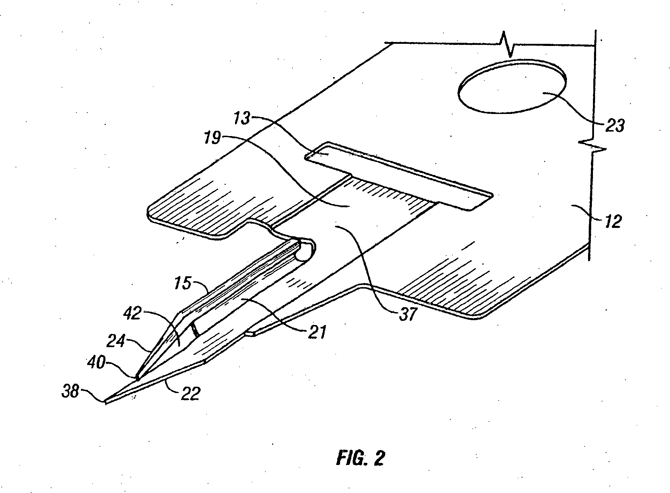

Figure 2 is a perspective view of lance 15 and the top layer of sensor strip 100 for use in the present invention. In Figure 2, the top layer of sensor strip 100 and lance 15 is formed from conductive substrate 12. In the embodiment illustrated in Figure 2, conductive substrate 12 includes vent hole 13 and registration hole 23. In Figure 2, lance 15 includes lancing element 22, separation element 24 and fill channel 21.

One embodiment of a lancing element and sensor strip suitable for use in the present invention may be described with reference to Figures 1 and 2. In the embodiment illustrated in Figures 1 and 2, sensor strip 100 includes first electrode contact 10, wherein first electrode contact 10 may be screen printed on an insulating substrate 18, and a second electrode contact 17, wherein second electrode contact 17 comprises a portion of conductive substrate 12 which is contiguous with reference electrode 37 and lance 15. In the embodiment of the lancing element and sensor strip illustrated in Figures 1 and 2, the orientation of first electrode contact 10 and second electrode contact 17 are arranged such that an analyte measurement meter, such as, for example, a glucose meter (not shown) can establish electrical contact with sensor strip 100. In the illustrated embodiment, first electrode contact 10 and second electrode contact 17 are arranged on the same side of insulating substrate 18 to facilitate contact of both electrodes at the distal end of sensor strip 100.

Sensor strip 100 is manufactured using adhesive layer 11 to attach insulating substrate 18 to conductive substrate 12. Adhesive layer 11 could be implemented in a number of ways, including using pressure sensitive material, heat activated material, or UV cured double sided adhesive material. Conductive substrate 12 may be, for example, a sheet of electrically conductive material such as gold or plated stainless steel. The geometry of conductive substrate 12 may be formed by, for example, stamping process or photo etching. In the embodiment illustrated in Figures 1 and 2, lance 15 may be manufactured as an integral part of conductive substrate 12. Vent hole 13, may be formed by, for example, punching through conductive substrate 12. Vent hole 13 is used to facilitate the transport of bodily fluid up lance 15 and across working electrode 36. Registration hole 23 may be formed during the stamping process of making conductive substrate 12.

In one embodiment of the invention, an analyte sensing layer may be, for example, a glucose sensing layer, including an enzyme, a buffer, and a redox mediator. An analyte sensing layer (not shown) may preferably be deposited on top of working electrode 36. Where an analyte sensing layer is used to detect the presence and concentration of glucose in a bodily fluid, at least a portion of glucose sensing layer dissolves in the bodily fluid and is used to convert the glucose concentration into an electrically measured parameter which is proportional to the glucose concentration in the sample.

In the embodiment illustrated in Figures 1 and 2, lance 15 has a distal and proximal end and the proximal end is integrated with reference electrode 37 and the distal end includes sharpened tip 38 at the distal end of lacing element 22. Lance 15 may be formed by the process of stamping or photo-etching a conductive metal sheet. Photo-etching lance 15 is also beneficial in facilitating the manufacture of a lancing element which has a sharp lancing element 22 and separation element 24. In a subsequent process step, lance 15, lancing element 22, and separation element 24 may be bent to form a "V" or "U" shaped channel geometry as shown in Figure 2. Fill channel 21 serves as a conduit from lancing element 22 and separation element 24 to working electrode 36 and reference electrode 37. In one embodiment of the present invention, the distal end of lacing element 22 and separation tip 40 of separation element 24 are offset by about 0.005 inches to 0.020 inches.

The design of lance 15 is adapted to more effectively cut skin due to a sharper leading point of lancing element 22. As illustrated in Figure 2, with separation tip 40 offset distally from sharpened tip 38 of element 22, the extreme distal end of lance 15 comprises only sharpened tip 38 which may be a very sharp point or edge to facilitate the initial incision as lancing element 22 enters the skin. In contrast, if lancing element 22 and separation element 24 were coincident, the leading point of lance 15 would include both sharpened tip 38 and separation tip 40 making the combination less sharp than the embodiment illustrated in Figure 2 and requiring more force to create the initial incision. The offset of sharpened tip 38 and separation tip 40 make lance 15 more manufacturable because it reduces the inherent alignment difficulties in bringing the sharp point of lancing element 22 and separation element 24 into alignment or contact with each other. The embodiment of the invention illustrated in Figures 1 and 2 is further beneficial because it enhances fluid egress by helping to spread and hold open the skin wound after the initial incision is made. In the embodiment illustrated in Figures 1 and 2, the lance 15 further includes reference electrode 37 and second electrode contact 17. Alternative embodiments may include forming all of the electrodes and electrode contacts on insulating substrate 18.

In the embodiment of the invention illustrated in Figure 2, lance 15 includes fill channel 21, wherein the seamless transition between the lancing element 22 and separation element 24; and fill channel 21 facilitates the flow of body fluid from the wound to working electrode 36. Additionally, the seamless transition between the lancing element 22, separation element 24 and fill channel 21 prevents the introduction of stop junctions which can impede the capillary flow rate of liquid samples. The unique geometry increases the likelihood that a liquid sample will sufficiently cover working electrode 36 and reference electrode 37 regardless of the height of the lance 15 above or below the skin wound, or even if lance 15 lies horizontally offset from the wound. In certain embodiments of the invention, sample can be applied to the side of lance 15 rather than just the proximal end of lance 15 which provides a user the option of dosing sample onto sensor strip 100 after a site has been lanced separately.

In the embodiment of the invention illustrated in Figure 2, the gap 42 between lancing element 22 and separation element 24 guides bodily fluids into fill channel 21. The increasing separation between lancing element 22 and separation element 24 as fluid moves distally towards fill channel 21 facilitates the drawing of fluid into fill channel 21 and from fill channel 21 to sensor strip 100. As gap 42 narrows towards a distal end of separation tip 40 of separation element 24, the surface tension between the bodily fluid in gap 42 and the walls of gap 42 increases, thus bodily fluid is drawn more readily into gap 42, and up into sensor strip 100. Gap 42 is also advantageous in that it facilitates the introduction of bodily fluids into fill channel 21 by facilitating the flow of bodily fluids positioned to the side of gap 42, thus enhancing the ways in which sensor strip 100 may be used to gather bodily fluids.

Fill channel 21 may facilitate the flow of bodily fluids by, for example, wicking or capillary action. In the embodiment illustrated in Figures 1 and 2, fill channel 21 has an open geometry which facilitates the wicking of viscous samples and provides for simpler manufacturing techniques when compared with closed capillary channels.

At least one of the separation element, lancing element and fill channel 21 is either coated with a surfactant coating or has undergone a hydrophilic surface treatment to increase the capillary flow force within the gap or the capillary force within fill channel 21, respectively. Additionally, the open geometry of fill channel 21 facilitates the wicking of sample because it prevents the formation of a vacuum block. In a closed channel geometry, a capillary inlet can become plugged if it is positioned too close to the wound or inside the wound preventing air from facilitating the flow of sample to the capillary. With the open geometry of fill channel 21, the proximal end of lance 15 can be positioned arbitrarily close to the source of the blood and allow for sufficient fill of sample. In this embodiment of the invention, the open geometry of fill channel 21 has the capacity to hold a larger sample volume than the minimum sample volume to cover reference electrode 37 and working electrode 36. The open geometry of fill channel 21 thus allows excess sample to accumulate along fill channel 21 which helps leave a cleaner wound site.

In the illustrated embodiment as shown in Figure 2, the geometry of reference electrode 37 may be formed during the stamping process which effectively embosses the surface of conductive substrate 12. The stamping process may provide the pressure needed to create a recess in conductive substrate 12 which can help define the distance between reference electrode 37 and working electrode 36. For certain applications of the described invention, it may be advantageous to control the distance between reference electrode 37 and working electrode 36 by embossing conductive substrate 12 instead of controlling the thickness of adhesive layer 11. For other applications of the described invention, it may also be advantageous to not emboss the conductive substrate 12 and use adhesive layer 11 to help define the geometry of reference electrode 37.

In the embodiment of sensor strip 100 illustrated in Figure 1, insulating substrate 18 consists of material such as polyester or ceramic on which a conductive material can be printed onto insulating substrate 18 through silk-screening, sputtering, or electro-less deposition. Conductive material deposited on insulating substrate 18 forms first electrode contact 10 and working electrode 36. Insulating layer 20 may be, for example, screen printed to form a boundary for first electrode contact 10 and working electrode 36.

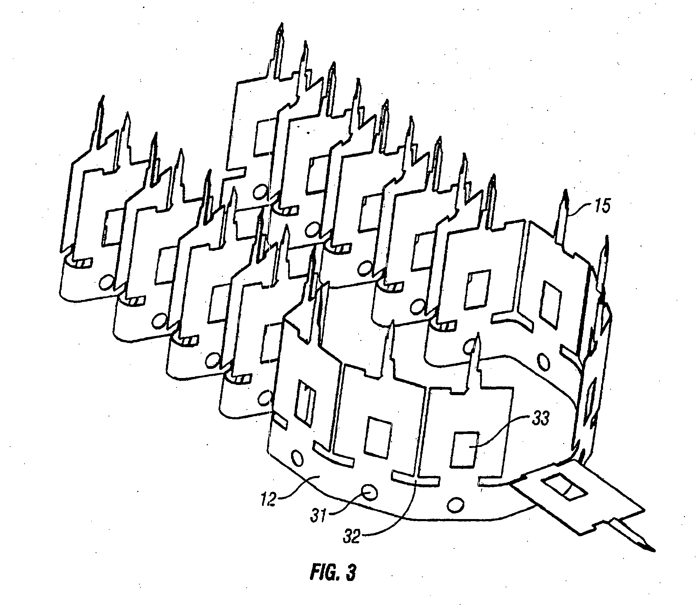

Figure 3 is a perspective view of another embodiment of the invention in which multiple strips form an array of sensors for use in a cartridge format. Such an array may be inserted into a meter (not shown) having strips dispensed in a serial manner, one by one. The format of this embodiment allows a row of strips to be folded in a manner similar to an accordion wherein several strips similar to sensor strip 100 in Figure 1 are attached together on an arrangement which facilitates their use in a cartridge. In Figure 3, conductive substrate 12 is stamped in a progressive manner to form lance 15 such that several of them are chained together in series. The stamping process of conductive substrate 12 forms index hole 31, neck 32 and contact hole 33.

In a further embodiment of the invention, a second electrode layer (not shown) comprising an adhesive layer and glucose sensing layer would be attached to conductive substrate 12 as illustrated in Figure 3. A contact area for a reference electrode for all of the strips within the array may be formed using a single area within conductive substrate 12. However, individual contacts must be made for working electrode 36 for all of the strips within the array. In the embodiment of this invention, index hole 31 is used to index the strip cartridge so that it can move a fresh strip to a test position. Neck 32 is punched in between 2 adjacent strips. The purpose of neck 32 is to facilitate the strip bending at the location of neck 32. In order for the strip to be expressed such that a user can apply blood, the strip is bent downward and neck 32 facilitates bending at a defined location. Contact hole 33 on conductive substrate 12 allows electrical contact to be made with a working electrode on an insulating substrate.

In a method of lancing in accordance with the present invention, a lance similar to the embodiments illustrated in Figures 1 through 3 is provided having a lancing element 22 with a sharpened tip 38, a separation element 24 having a separation tip 40 is positioned proximal to sharpened tip 38. In one embodiment of the invention the separation tip 40 may be positioned between approximately .005 inches and .020 inches proximal to sharpened tip 38. A method according to the present invention further includes the step of providing a connector connecting the proximal end of lancing element 22 to the proximal end of separation element 24 wherein the connector forms a fill channel 21 extending from the proximal end of lancing element 22 and the proximal end of separation element 24 to a working electrode 36 of sensor strip 100. The method further including the steps of inserting the lancing element into skin to form an incision, inserting the separation element 24 to further open the incision and maintaining the position of the lancing element 22 and the separation element 24 in the incision while blood or other bodily fluids are drawn into a gap 42 between the lancing element 22 and separation element 24. The method further comprising the step of drawing the bodily fluids from gap 42 into fill channel 21.

A lance 15 constructed in accordance with the present invention is beneficial due to the seamless transition between the tip section and the capillary section, and because the tip itself is a type of capillary. The unique construction of this design better insures that bodily fluids enter the fill channel 21 regardless of the height of the tip above or below the skin wound, or even if the tip lies horizontally offset from the wound, where the lance acts as a conduit for the bodily fluids.

A sensor strip 100 constructed according to the present invention is more easily by manufactured than a closed channel sensor strip. Such a strip may be manufactured by, for example, injection molding, embossing, or chemical etching, or even simple machining. While the capillary force of an open channel may be weaker than a comparable closed channel, the weakness can be overcome with the use of, for example, hydrophilic surface treatments or surfactant coatings including: Tween-80, a product of Sigma Chemical Co., St. Louis, Mo.; Aerosol OT a product of Cytec Industries, West Paterson, New Jersey; JBR-515, a product of Jeneil Biosurfactant Company of Saukville, Wisconsin; and Niaproof a product of Sigma Chemical Co., St. Louis, Mo.

A sensor strip 100 constructed according to the present invention may have improved transfer properties because the invention described herein prevents the creation of a vacuum block in fill channel 21 that would prevent fluid from moving through the fill channel 21 and onto the measurement pad. With a closed channel capillary, the inlet must be positioned or designed to ensure that air is not prevented from freely entering the capillary during transfer into the measurement area. Thus, in a closed channel system, if the inlet is positioned too close to the wound or even inside it, flow may be disrupted or stopped. With the open channel of a sensor strip designed in accordance with the present invention, however, the inlet to the channel can be positioned arbitrarily close to the source of the blood.

Another advantage of a strip in accordance with the present invention including an open channel is that such a strip has the capacity to hold a larger volume of fluid than the minimum required to fill and initiate transfer into the measurement pad. One embodiment of the present invention the minimum volume required to fill the lance such that the column of fluid reaches the measurement pad is approximately 230 nL. However, lancing may produce quantities which are greater than 230nL. Because of the open channel form in the present invention, the excess blood that is presented to the lance will continue to accumulate along the lance channel, forming a bulging drop of blood. This property is useful in that it clears away excess blood from the skin, leaving a cleaner lance wound.

Another advantage of the open channel design in accordance with the present invention is that a drop of fluid can be applied to the side of the lance rather than just at the tip of the lance (i.e. in a closed channel there is a distinct area where fluid must be presented to be drawn into the capillary. Manual application of blood might be required if the blood comes from a site that has been lanced separately. Thus, using a sensor strip designed in accordance with the present invention, provides the option of 'side' filling increases the user's options.

In one embodiment of the present invention, the stamped metal of conductive substrate 12 could also serve as a working or counter electrode. A unique aspect of the sheet metal design used in the present invention is the fact that it also allows the assembly to be constructed with first electrical contact 10 and second electrode contact 17 on the same side of the strip. This greatly simplifies the requirements for mating contacts on a meter because conductive substrate 12 comprises a solid conductor allowing electrical contact to be established from both the top and bottom side of conductive substrate 12, wherein the top side of conductive substrate 12 is on the same side as second electrical contact 17 and the bottom side of conductive substrate is on the same side as reference electrode 37.

On a conventionally constructed electrochemical strips using a facing electrode arrangement where both working and reference electrodes are printed or applied onto an insulating substrate, the electrical contacts must be positioned on opposites sides of the strip making the meter contacts more complex. If reference electrode 37 was printed or applied onto an insulating substrate, conductive substrate 12 would be insulated on the top side preventing electrical connection to be established from the top side. It could be possible to establish electrical connection from the top side if there was a partial removal of insulation from conductive substrate 12, however, this would add additional complexity to the manufacturing of the strip.

Finally, because sheet metal forming can be done as a progressive die stamping, in a strip designed in accordance with the present invention with individual lances chained together in series, it could be possible to construct an array of test sensors with a single, common reference thus requiring just one contact.

It will be recognized that equivalent structures may be substituted for the structured illustrated and described herein and that the described embodiment of the invention is not the only structure which may be employed to implement the claimed invention. In addition, it should be understood that every structure described above has a function and such structure can be referred to as a means for performing that function.

While preferred embodiments of the present invention have been shown and described herein, it will be obvious to those skilled in the art that such embodiments are provided by way of example only. Numerous variations, changes, and substitutions will now occur to those skilled in the art without departing from the invention.

It should be understood that various alternatives to the embodiments of the invention described herein may be employed in practising the invention. It is intended that the following claims define the scope of the invention and that methods and structures within the scope of these claims and their equivalents be covered thereby.

## Claims

1. A lance (15), for lancing skin, comprising:
a lancing element (22) having a first sharpened end point (38);
a separation element (24) having a second end point (40) positioned adjacent said lancing element (22);
a connector, connecting a proximal portion of said lancing element (22) to a proximal portion of said separation element (24), said connector forming a channel (21);
wherein:
said lancing element (22) is adapted for insertion to form an incision;
said separation element (24) is adapted for insertion to further open said incision;
said lancing element (22) and said separation element (24) are adapted to be maintained in said incision while bodily fluids are drawn into a space between said elements; **characterised in that**
at least one of said lancing element, said separation element and said channel comprises either (i) a surfactant coating, or (ii) a hydrophilic surface treatment; and
**in that**
said separation element (24) is positioned adjacent said lancing element (22) such that an increasing separation between the lancing element and the separation element forms a gap (42) between said lancing element (22) and said separation element (24), said gap increasing in size towards the channel (21) and narrowing towards the second end point (40) of the separation element (24).

2. The lance (15) of Claim 1, which is further adapted such that said bodily fluids are drawn from said space into said channel (21).

3. The lance (15) of Claim 1 or Claim 2, further comprising a sensor strip (100) attached to a proximal portion of said connector.

4. The lance (15) of Claim 3 when dependent on Claim 2, which is further adapted such that said bodily fluids are drawn from said channel (21) into said sensor strip (100).

5. The lance (15) of any one of Claims 1 to 4, which is adapted such that, on removing said lancing element (22) from said incision, said bodily fluids are drawn into said gap (42).

## Patentansprüche

1. Lanzette (15) zum Einstechen in Haut, umfassend:
ein Lanzettenelement (22) mit einem ersten angespitzten Endpunkt (38);
ein Trennelement (24) mit einem zweiten Endpunkt (40),
der neben dem Lanzettenelement (22) angeordnet ist,
ein Verbindungsglied, das einen proximalen Abschnitt des Lanzettenelements (22) mit einem proximalen Abschnitt des Trennelements (24) verbindet, wobei das Verbindungsglied einen Kanal (21) bildet;
worin:
das Lanzettenelement (22) zur Bildung eines Einschnitts eingeführt wird;
wobei das Trennelement (24) zur weiteren Öffnung des Einschnitts eingeführt wird;
wobei das Lanzettenelement (22) und das Trennelement (24) in dem Einschnitt gehalten werden, während Körperflüssigkeiten in einen Raum zwischen den Elementen gezogen werden; **dadurch gekennzeichnet, dass** zumindest das Lanzettenelement, das Trennelement und/oder der Kanal (i) eine Tensidbeschichtung oder (ii) eine hydrophile Oberflächenbehandlung umfasst; und dass
das Trennelement (24) neben dem Lanzettenelement (22) angeordnet ist, so dass ein zunehmender Abstand zwischen dem Lanzettenelement und dem Trennelement einen Spalt (42) zwischen dem Lanzettenelement (22) und dem Trennelement (24) bildet, wobei der Spalt zum Kanal (21) hin größer wird und zum zweiten Endpunkt (40) des Trennelements (24) kleiner wird.

2. Lanzette (15) nach Anspruch 1, die ferner so ausgelegt ist, dass die Körperflüssigkeiten aus dem Raum in den Kanal (21) gezogen werden.

3. Lanzette (15) nach Anspruch 1 oder Anspruch 2, ferner umfassend einen Sensorstreifen (100), der an einem proximalen Abschnitt des Verbindungsglieds befestigt ist.

4. Lanzette (15) nach Anspruch 3, in Abhängigkeit von Anspruch 2, die ferner so ausgelegt ist, dass die Körperflüssigkeiten aus dem Kanal (21) in den Sensorstreifen (100) gezogen werden.

5. Lanzette (15) nach einem der Ansprüche 1 bis 4, die so ausgelegt ist, dass bei Entfernung des Lanzettenelements (22) aus dem Einschnitt die Körperflüssigkeiten in den Spalt (42) gezogen werden.

## Revendications

1. Lancette (15), pour perforer une peau, comprenant:
un élément de perforation (22) présentant une première pointe d'extrémité tranchante (38);
un élément de séparation (24) présentant une deuxième pointe d'extrémité (40) positionnée à proximité dudit élément de perforation (22);
un connecteur, qui relie une extrémité proximale dudit élément de perforation (22) à une partie proximale dudit élément de séparation (24), ledit connecteur formant un canal (21),
dans laquelle:
ledit élément de perforation (22) est adapté pour être inséré dans le but de pratiquer une incision;
ledit élément de séparation (24) est adapté pour être inséré pour ouvrir davantage ladite incision;
ledit élément de perforation (22) et ledit élément de séparation (24) sont adaptés pour être maintenus dans ladite incision pendant que des fluides corporels sont aspirés dans un espace entre lesdits éléments,
**caractérisée en ce que**:
au moins un élément parmi ledit élément de perforation, ledit élément de séparation et ledit canal comprend (i) soit un revêtement tensioactif, soit (ii) un traitement de surface hydrophile; et **en ce que**:
ledit élément de séparation (24) est positionné à proximité dudit élément de perforation (22), de telle sorte qu'une séparation grandissante entre l'élément de perforation et l'élément de séparation forme un espace (42) entre ledit élément de perforation (22) et ledit élément de séparation (24), la taille dudit espace grandissant en direction du canal (21) et rétrécissant en direction de la deuxième pointe d'extrémité (40) de l'élément de séparation (24).

2. Lancette (15) selon la revendication 1, qui est en outre adaptée de telle sorte que lesdits fluides corporels soient aspirés à partir dudit espace dans ledit canal (21).

3. Lancette (15) selon la revendication 1 ou la revendication 2, comprenant en outre une bande de capteur (100) qui est attachée à une partie proximale dudit connecteur.

4. Lancette (15) selon la revendication 3 lorsqu'elle dépend de la revendication 2, qui est en outre adaptée de telle sorte que lesdits fluides corporels soient aspirés à partir dudit canal (21) dans ladite bande de capteur (100).

5. Lancette (15) selon l'une quelconque des revendications 1 à 4, qui est adaptée de telle sorte que, lors de l'enlèvement dudit élément de perforation (22) hors de ladite incision, lesdits fluides corporels soient aspirés dans ledit espace (42).
